# EUROPEAN PATENT APPLICATION

(11) **EP 1 533 002 A1**
(43) Date of publication of application: **25.05.2005**
(21) Application number: 04256763.6
(22) Date of filing: 02.11.2004
(51) Int. Cl.: A61N 2/06, A61N 2/02

(54) **Apparatus and method for controlling blood circulation using a magnetic field**

(30) Priority: 18.11.2003 KR 2003081740
(71) Applicant: Samsung Electronics Co., Ltd., Suwon-city, Gyeonggi-do (KR); Koo, Han-seo, Suseong-gu, Daegu-si (KR)
(72) Inventor: Jang, Woo-young, Seoul (KR); Shin, Sang-hoon, Bundang-gu Seongnam-si Gyeonggi-do (KR); Park, Jae-chan, Yuseong-gu Daejeon-si (KR); Gi, Ho-seong, Buan-gun Jeollabuk-do (KR)
(74) Representative: Greene, Simon Kenneth

(57) **Abstract**

In an apparatus and method for controlling blood circulation in a living body using a magnetic field to improve blood circulation, the apparatus includes a magnetic field generating unit for generating a magnetic field that is oriented in a direction of blood flow and an affixing unit for affixing the magnetic field generating unit to the living body.

## Description

The present invention relates to an apparatus and method for controlling blood circulation using a magnetic field. More particularly, the present invention relates to an apparatus and method for controlling blood circulation of a living body by promoting the blood circulation using a bipolar magnetic field.

Generally, blood circulation or blood flow in a living body directly or indirectly affects the health of that living body. For example, if a person has poor blood circulation in the stomach and intestines, the person has a digestive tract disorder. If a person has poor or inefficient blood circulation in the limbs, the person experiences pains in the limbs. If a person has poor or irregular blood circulation in a joint, e.g., a knee, the person suffers from arthritis. If a person has poor circulation in the heart, the person has a cardiac disorder. If a person has poor or weak blood circulation to the head, the person has headaches or degraded brain capabilities. If a person has poor blood circulation in the lungs, the person suffers from lung troubles. Further, a serious problem of blood circulation could cause cerebral infarction, cerebral apoplexy, or cerebral hemorrhage. When blood does not circulate smoothly in a portion of the body, oxygen and nutritive substances are not sufficiently supplied to the portion and waste materials resulting from metabolism are not properly removed. Resultantly, a person may suffer from pain, afflictions, or diseases.

When a variety of blood circulation disorders occur in a human body, a person may perform several actions in order to improve the blood circulation. For example, a person may warm up a portion of the body or the whole body, may stretch to improve an elasticity of muscles, may exercise, may take blood circulation improving medicine, may transfer waste materials from one portion of the body to other portions of the body by pressing specific areas of the body with the fingers, or may directly extract dead white corpuscles or dead red cells from portions of the body.

In recent years, there have been attempts to treat pain or other afflictions by improving blood circulation using a magnetic field. For example, some conventional methods have taught treating diseases and inflammations of a living body by generating a unipolar magnetic field by attaching a unipolar magnet to the body. Additionally, conventional devices have included north poles and south poles on opposite sides of a device and methods of treating ailments or diseases by maintaining the north pole surface in contact with a first ailing body part and the south pole surface in contact with a second ailing body part.

However, these conventional methods or devices using bipolar magnets still concentrate on stimulating the skin of a living body using single poles irrespective of the directionality of blood circulation.

Additional references and research has shown that human diseases, such as infantile paralysis, may be alleviated or cured using a magnetic field.

These results show positive effects of applying a magnetic field to improve blood circulation and alleviate pains. However, since these methods utilize a magnetic field that is applied to a living body without regard for the directionality of blood circulation and do not experimentally demonstrate the effects of the magnetic field, their reliability is low.

According to the invention, there is provided an apparatus for controlling blood circulation in a living body using a magnetic field, the apparatus including a magnetic field generating unit for generating a magnetic field that is oriented in a direction of blood flow and an affixing unit for affixing the magnetic field generating unit to the living body.

The direction of blood flow may be a direction of arterial blood flow from a heart of the living body. The magnetic field generating unit may be a permanent magnet or an electromagnet. The magnetic field generating unit may generate a magnetic field between about 2000 to 3000 gauss.

The magnetic field generating unit may include an affixing main body, a plurality of first affixing members combined with the affixing main body, a plurality of second affixing members combined with the affixing main body, each of the plurality of second affixing members being operable to combine with a corresponding one of the plurality of first affixing members to affix the apparatus to the living body, a first magnetic field generating member mounted on the affixing main body and having a north pole that faces blood vessels of the living body, and a second magnetic field generating member mounted on the affixing main body and having a south pole that faces blood vessels of the living body.

The first affixing members may be selected from the group consisting of VELCRO®, a button, and adhesive tape.

The magnetic field generating unit may include an affixing main body, a plurality of first affixing members combined with the affixing main body, a plurality of second affixing members combined with the affixing main body, each of the plurality of second affixing members being operable to combine with a corresponding one of the plurality of first affixing members to secure the apparatus, and a plurality of magnetic field generating members mounted on the affixing main body, wherein each of the plurality of magnetic field generating members are disposed in a direction to orient a magnetic field thereof in a direction of blood flow, each of the plurality of magnetic field generating members being spaced a predetermined distance apart from each other.

Each of the magnetic field generating members may have a north pole that faces the blood vessels of the living body and a south pole that faces the blood vessels of the living body, and the north pole may be disposed nearer to a heart of the living body than the south pole.

The magnetic field generating unit may include a pair of band-type magnetic field generators, one of which is disposed near a heart of the living body and has a north pole that faces the blood vessels of the living body near the heart and the other of which is disposed relatively farther away from the heart of the living body and has a south pole that faces the blood vessels of the living body away from the heart. Each of the pair of band-type magnetic field generators may encompass the living body.

According to another aspect of the invention, there is provided a method of controlling blood circulation using a magnetic field including generating a magnetic field in blood vessels of a living body and controlling a direction of the magnetic field to orient a direction of the magnetic field in a direction of blood flow.

The magnetic field generated may be about 2,000 to 3,000 gauss. The direction of blood flow may be a direction of arterial blood flow from a heart of the living body.

The present invention thus provides an apparatus and method for controlling blood circulation of a living body by promoting blood circulation using a bipolar magnetic field that is able to improve blood circulation in the living body, and that avoids causing adverse side effects on the living body.

The above and other features and advantages of the present invention will become more apparent to those of ordinary skill in the art by describing in detail exemplary embodiments thereof with reference to the attached drawings in which:
FIG. 1 illustrates an apparatus for controlling blood circulation in a living body using a magnetic field according to an embodiment of the present invention, the apparatus being shown attached to a leg of a person;
FIG. 2 illustrates the apparatus for controlling blood circulation in a living body using a magnetic field according to an embodiment of the present invention, the apparatus being shown attached to a forearm of a person;
FIG. 3A illustrates a perspective view of an apparatus for controlling blood circulation in a living body using a magnetic field according to an embodiment of the present invention;
FIG. 3B illustrates a perspective view of an apparatus for controlling blood circulation in a living body using a magnetic field according to another embodiment of the present invention;
FIG. 4 illustrates a cross-sectional view of a magnetic field generating member showing that a magnetic field generated from the apparatus shown in FIG. 3A or 3B is oriented in a direction of blood flow;
FIG. 5 is a conceptual diagram for explaining the direction of a magnetic field generated from the magnetic field generating member shown in FIG. 4; and
FIG. 6 is a graph showing results of an experiment on thirty (30) examinees using real and false magnets for explaining an effect of the apparatus according to an embodiment of the present invention.

The present invention will now be described more fully hereinafter with reference to the accompanying drawings, in which exemplary embodiments of the invention are shown. The invention may, however, be embodied in different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

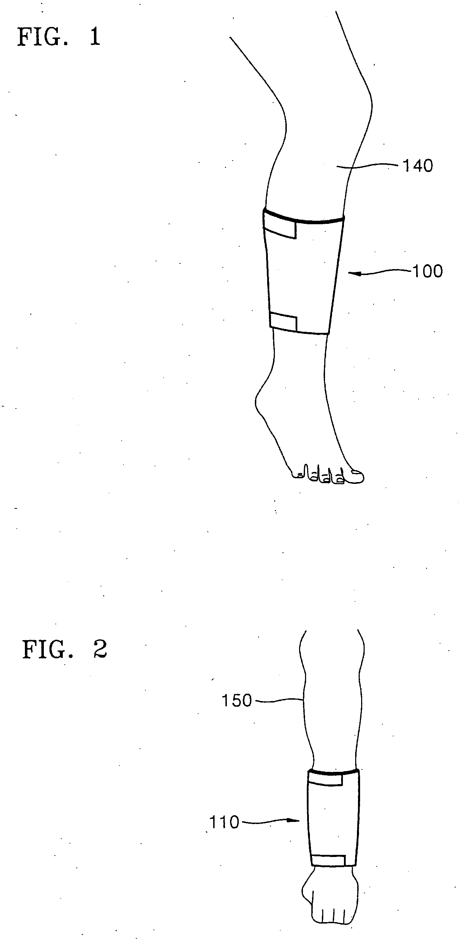

FIG. 1 illustrates an apparatus 100 for controlling blood circulation in a living body using a magnetic field according to an embodiment of the present invention, which is attached to a leg 140 of a person. 'FIG. 2 illustrates the apparatus 100 being attached to a forearm 150 of a person.

Although the apparatus 100 is shown attached to the leg 140 or forearm 150 in FIGS. 1 and 2, respectively, an apparatus according to an embodiment of the present embodiment may be attached to other portions of a human body. By way of further alternative, an apparatus according to an embodiment of the present embodiment may be attached to a portion of a body of an animal, e.g., a dog, a pig, or a cow.

The apparatus 100 improves blood circulation of any portion of the body by adjusting a direction and an intensity of a magnetic field or sizes of poles of an electromagnet. For example, the apparatus 100 can be utilized to alleviate diseases, such as local hardening of the arteries, a local glycosuric disorder of the blood vessels, and varices caused by venous obstruction, and generally to improve the blood circulation of a person.

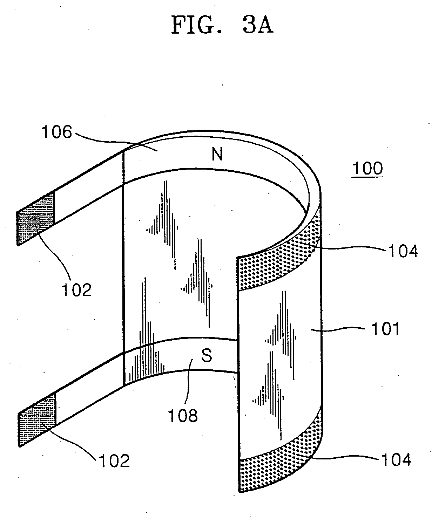

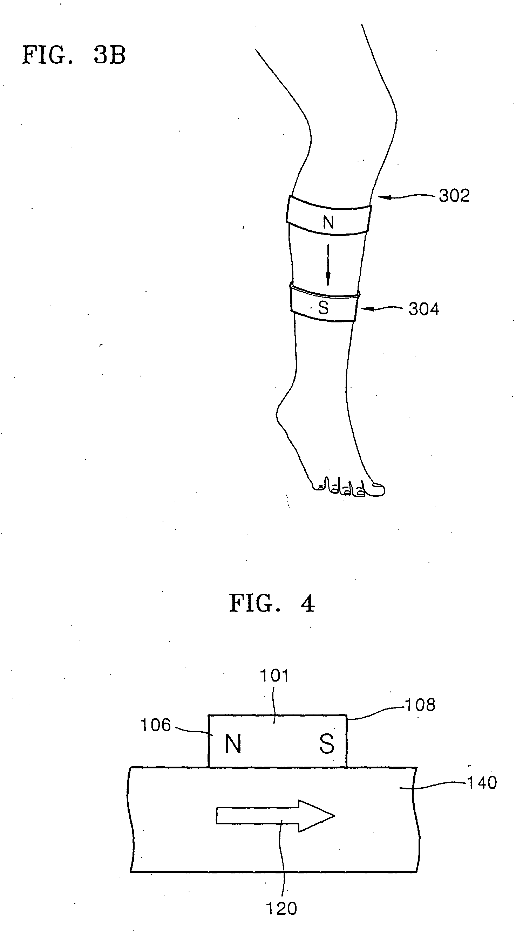

FIGS. 3A and 3B illustrate perspective views of various embodiments of the apparatus for controlling blood flow circulation in a living body according to the present invention. Either of these embodiments may be used as the apparatus 100 shown in FIGS. 1 or 2.

FIG. 3A illustrates the apparatus 100 according to an embodiment of the present invention.

Referring to FIG. 3, the apparatus 100 includes an affixing main body 101, a plurality of first affixing members 102, a plurality of second affixing members 104, a first magnetic field generating member 106, and a second magnetic field generating member 108. Although FIG. 3 illustrates two magnetic field generating members, i.e., a first and a second magnetic field generating member 106 and 108, more than two, i.e., a plurality of magnetic field generating members may be provided. In a case of a plurality of magnetic field generating members being provided, each of the plurality of magnetic field generating members is spaced a predetermined distance apart from each other.

The first affixing members 102 each include an adhesive portion, such as a VELCRO®, a button, or an adhesive tape. The second affixing members 104 are each capable of being combined with a corresponding one of the first affixing members 102 to affix the apparatus of the present invention to the living body, e.g., in a VELCRO® assembly, a button and hole assembly, or a surface to receive adhesive tape.

The first magnetic field generating member 106 is mounted on the affixing main body 101 and has a north pole that faces blood vessels of a living body. The second magnetic field generating member 108 is mounted on the affixing main body 101 and has a south pole that faces blood vessels of the living body. The first and second magnetic field generating members 106 and 108 each have a band shape. To attach the apparatus 100 to a wrist or calf of a person, the first and second magnetic field generating members 106 and 108 each have a sufficient length so that a combination of the first and second affixing members 102 and 104 and the first and second magnetic field generating members 106 and 108 encompass the wrist or calf of the person.

To orient a magnetic field in a direction of blood flow, a lengthwise direction of each of the first and second magnetic field generating members 106 and 108, which may be integrally formed, is oriented perpendicular to the direction of blood flow. Thus, since the magnetic field directed from the first magnetic field generating member 106 flows toward the second magnetic field generating member 108, the magnetic field is oriented in the direction of blood flow.

Although it is described herein that the first and second magnetic field generating members 106 and 108 are fixed to a living body using wrappers, i.e., the first and second affixing members 102 and 104, the first and second magnetic field generating members 106 and 108 may alternatively be attached to an armrest of a car seat or a sofa.

Preferably, the direction of blood flow in which the magnetic field is oriented is a direction in which the arterial blood flows. Here, the direction of arterial blood flow is defined as a direction in which the blood flows away from the heart of a living body toward terminal portions of the living body, such as a hand or a foot.

FIG. 3B illustrates a perspective view of an apparatus 100 according to another embodiment of the present invention.

Unlike the embodiment shown in FIG. 3A, the apparatus 100 shown in FIG. 3B includes a pair of magnetic field generating units 302 and 304, each of which is formed as a band encompassing a portion of a living body to be treated.

By using the magnetic field generating units 302 and 304, the apparatus 100 of FIG. 3B has the same effect as that of FIG. 3A and can apply a magnetic field to a living body in the same direction as that of blood flow.

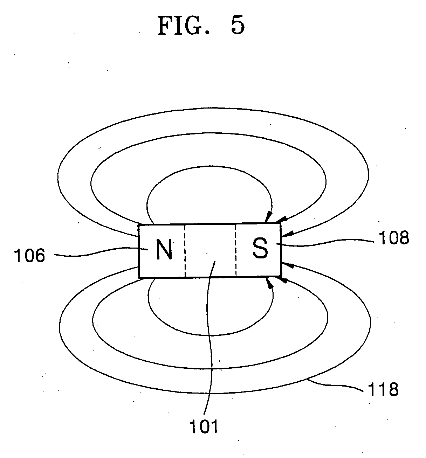

FIG. 4 illustrates a cross-sectional view of a magnetic field generating member showing that a magnetic field generated from the apparatus shown in FIG. 3A or 3B is oriented in a direction of blood flow. FIG. 5 is a conceptual diagram for explaining the direction of a magnetic field generated from the magnetic field generating member shown in FIG. 4.

In use, the arteries of a living body, such as the leg 140, where blood circulation requires improvement, are initially selected. Then, north and south poles of a magnetic field, generated from the first and second magnetic field generating members 106 and 108 mounted on the affixing main body 101 of the apparatus 100, are oriented in a direction 120 of blood circulation. Thus, a direction 118 of the magnetic field is identical to the direction 120 of blood circulation.

In FIGS. 3A, 3B, 4, and 5, the magnetic field generating members 106 and 108 are each formed using a permanent magnet. However, as long as blood circulation is improved by orienting the magnetic field in the direction of blood circulation, each of the magnetic field generating members 106 and 108 may be an electromagnet having the same effect as a permanent magnet. Thus, the user can arbitrarily adjust an intensity and direction of a magnetic field appropriate for an individual user.

Hereinafter, an operational principle of the apparatus for controlling blood circulation in a living body using a magnetic field according to the present invention will be described.

The apparatus 100 operates based on the principle that if a magnetic field is applied to a living body and a direction of the magnetic field 118 is oriented in the direction 120 of blood circulation, blood circulation is improved.

Specifically, blood circulation is produced as cardiac muscles compress and contract blood in the heart in response to an electrical signal to the heart. Thus, blood flows from the heart, through the arteries, into all of the organs and limbs of a living body. The blood provides capillary vessels with oxygen and nutritive substances, such as protein, which are required for basic metabolism at terminals of the arteries. Waste materials result from the metabolism in the capillary vessels. Blood after metabolism and the waste materials from that metabolism flow through the veins and return to the heart. This circulation of blood continues until a living body dies.

Blood flow in the arteries is driven by a blood pressure from cardiac movements. Blood flow in the veins is driven by contractions of vessel muscles surrounding the veins. Poor blood circulation in either the arteries or the veins causes various complications.

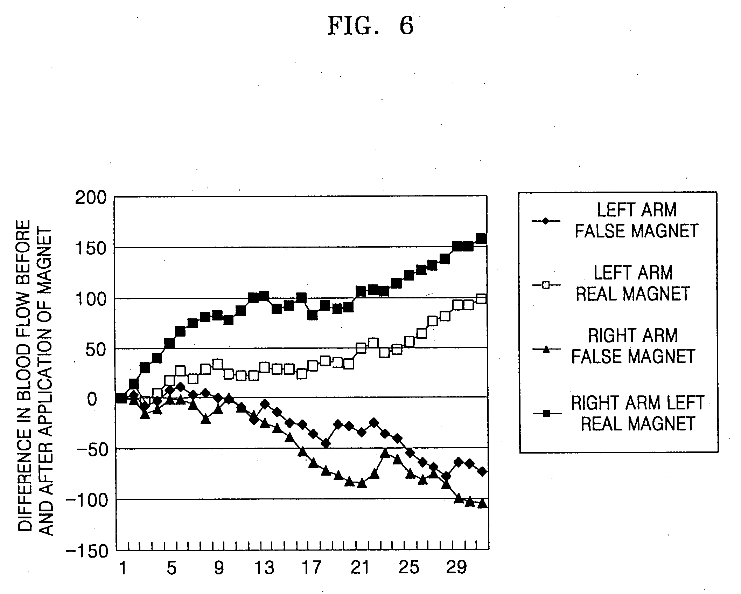

FIG. 6 is a graph showing results of an experiment on thirty (30) examinees using real and false magnets for explaining an effect of the apparatus according to an embodiment of the present invention.

In FIG. 6, the x-axis denotes the number of examinees, and the y-axis denotes an accumulated value of values obtained by subtracting an amount of blood flow measured before application of a magnet, real or false, from an amount of blood flow measured thereafter. For example, in a case of a first examinee, if an amount of blood flow measured before application of a magnet is equal to an amount of blood flow measured thereafter, a y-axis value is zero (0).

Referring to FIG. 6, when the left and right arms of thirty (30) examinees were stimulated by randomly applying a real magnet and a false magnet under the same conditions, the graph shows a variation of the value obtained by subtracting the amount of blood flow measured before stimulation from the amount of blood flow measured thereafter. The experiment was performed under the same conditions throughout. In the experiment, a real magnet was a magnet having a diameter of about 1.5 cm and a length of about 1.5 cm with about 3,000 gauss and a false magnet was a nonmagnetic body having a same size and shape as the real magnet. The magnets were each applied for about twenty (20) minutes.

In general, the amount of blood flow in a living body slowly decreases when the living body is in stable state. As shown in FIG. 6, when a real magnet was applied, however, as the number of examinees increased, an accumulated value of differences between the amounts of blood flow before and after stimulation increased, i.e., after the real magnet was applied, the amount of blood flow increased. However, when the false magnet was utilized, the amount of blood flow relatively decreased, since the false magnet has no effect on blood flow. As a result, the real magnet generally increased the amount of blood flow, while the false magnet decreased the amount of blood flow.

Further, to provide high reliability, after a magnetic field had either been applied in a direction of blood flow using the real magnet or not applied at all using the false magnet, the amount of blood flow was measured. As a result, it can be statistically confirmed that the real magnet differed from the false magnet and increased the amount of blood flow as compared to the false magnet.

As described above, the apparatus and method of the embodiments of the present invention improve blood circulation by applying a magnetic field to an afflicted region of a living body in a direction of blood flow as opposed to conventional treating methods using a magnetic field.

Further, conventional methods of improving blood circulation using medicines or mechanical means are not able to completely avoid side effects caused by artificial stimulation to a living body. The present invention, however, uses only the directionality of a magnetic field of about 2,000 to 3,000 gauss, which has no adverse effect on a user.

This effect is confirmed by the foregoing experiment, which takes into account a placebo effect by measuring effects of both real and false magnets. Thus, it can be seen that the real magnet increased the amount of blood flow unlike the false magnet, which decreased the same.

Further, in the present invention, a magnetic field can be applied to patients afflicted with arterial and venous disorders and ailments in a direction in which the blood flows through blood vessels. As a result, the blood in a body of a user can circulate smoothly, thus improving the user's health.

Exemplary embodiments of the present invention have been disclosed herein and, although specific terms are employed, they are used and are to be interpreted in a generic and descriptive sense only and not for purpose of limitation. Accordingly, it will be understood by those of ordinary skill in the art that various changes in form and details may be made without departing from the scope of the present invention as set forth in the following claims.

## Claims

1. An apparatus for controlling blood circulation in a living body using a magnetic field, the apparatus comprising:
a magnetic field generating unit for generating a magnetic field that is oriented in a direction of blood flow; and
an affixing unit for affixing the magnetic field generating unit to the living body adapted to orient the magnetic field in a direction of blood flow.

2. The apparatus as claimed in claim 1, wherein the direction of blood flow is a direction of arterial blood flow from a heart of the living body.

3. The apparatus as claimed in claim 1 or 2, wherein the magnetic field generating unit is a permanent magnet.

4. The apparatus as claimed in claim 1 or 2, wherein the magnetic field generating unit is an electromagnet.

5. The apparatus as claimed in any preceding claim, wherein the magnetic field generating unit generates a magnetic field between about 2000 to 3000 gauss.

6. The apparatus as claimed in any preceding claim, wherein the magnetic field generating unit comprises:
a main body;
a plurality of first affixing members combined with the main body;
a plurality of second affixing members combined with the main body, each of the plurality of second affixing members being operable to combine with a corresponding one of the plurality of first affixing members for affixing the apparatus to the living body;
a first magnetic field generating member mounted on the main body and having a north pole for facing blood vessels of the living body; and
a second magnetic field generating member mounted on the main body and having a south pole for facing blood vessels of the living body.

7. The apparatus as claimed in any one of claims 1 to 5, wherein the magnetic field generating unit comprises:
a main body;
a plurality of first affixing members combined with the main body;
a plurality of second affixing members combined with the main body, each of the plurality of second affixing members being operable to combine with a corresponding one of the plurality of first affixing members for securing the apparatus; and
a plurality of magnetic field generating members mounted on the main body,
wherein each of the plurality of magnetic field generating members are disposed in a direction for orienting a magnetic field thereof in a direction of blood flow of the living body, each of the plurality of magnetic field generating members being spaced a predetermined distance apart from each other.

8. The apparatus as claimed in claim 7, wherein each of the magnetic field generating members has a north pole for facing the blood vessels of the living body and a south pole for facing the blood vessels of the living body, and wherein the north pole is for disposal nearer to a heart of the living body than the south pole.

9. The apparatus as claimed in any one of claims 6 to 8, wherein the first affixing members are selected from VELCRO®, a button, and adhesive tape.

10. The apparatus as claimed in any one of claims 1 to 5, wherein the magnetic field generating unit comprises a pair of band-type magnetic field generators, one of which is for disposal near a heart of the living body and has a north pole that is for facing the blood vessels of the living body near the heart and the other of which is for disposal relatively farther away from the heart of the living body and has a south pole that is for facing the blood vessels of the living body away from the heart.

11. The apparatus as claimed in claim 10, wherein each of the pair of band-type magnetic field generators are for encompassing the living body.

12. A method of controlling blood circulation using a magnetic field, comprising:
generating a magnetic field in blood vessels of a living body; and
controlling a direction of the magnetic field to orient a direction of the magnetic field in a direction of blood flow.

13. The method as claimed in claim 12, wherein the magnetic field generated is about 2,000 to 3,000 gauss.

14. The method as claimed in claim 12 or 13, wherein the direction of blood flow is a direction of arterial blood flow from a heart of the living body.

15. An apparatus for improving blood circulation in a living body, the apparatus comprising:
a magnetic field generating unit adapted to generate a magnetic field; and
an affixing unit adapted for fixing the magnetic field generating unit to the living body,
wherein the affixing unit is adapted such that in use, the magnetic field of the magnetic field generating unit is substantially orientated in a direction of arterial blood flow of the living body.
